# EUROPÄISCHE PATENTSCHRIFT

(11) **EP 4 169 547 B1**
(45) Veröffentlichungstag und Bekanntmachung des Hinweises auf die Patenterteilung: **02.10.2024**
(21) Anmeldenummer: 21204488.7
(22) Anmeldetag: 25.10.2021
(51) Int. Cl.: A61M 5/142

(54) **VERBESSERTE VERABREICHUNGSVORRICHTUNG**
IMPROVED ADMINISTRATION DEVICE
DISPOSITIF D'ADMINISTRATION AMÉLIORÉ

(43) Veröffentlichungstag der Anmeldung: 26.04.2023
(73) Patentinhaber: TecMed AG, 3400 Burgdorf (CH)
(72) Erfinder: Streit, Ursina, 3422 Kirchberg (CH); Baumert, Jan, 3455 Grünen (CH); Buri, Thomas, 3400 Burgdorf (CH); Hanimann, Michael, 3012 Bern (CH); Margot, Roland, 3076 Worb (CH); Steiner, Fabian, 3400 Burgdorf (CH); Hostettler, Patrick, 3415 Hasle (CH); Bosshard, Simon Martin, 3006 Bern (CH); Brügger, Martin, 3065 Bolligen (CH)
(74) Vertreter: Meier Obertüfer, Jürg

(56) Entgegenhaltungen:
- EP-A1- 3 110 475
- US-A1- 2008 051 711

## Beschreibung

### TECHNISCHES GEBIET

Die vorliegende Erfindung betrifft das Gebiet der Verabreichungsgeräte und Verabreichungsvorrichtungen für fluide Medikamente, insbesondere Infusions- oder Injektionsgeräte. Speziell betrifft die vorliegende Erfindung Verabreichungsgeräte mit beweglichen Kolbenstangen.

### HINTERGRUND DER ERFINDUNG

Verabreichungsgeräte für fluide, insbesondere flüssige Medikamente sind in einer grossen Vielfalt bekannt. Von einfachen Einwegspritzen über Injektionspens bis hin zu Infusionsgeräten mit ausgeklügelten Funktionen. Insbesondere bei wiederverwendbaren Verabreichungsgeräten ist es wichtig für die Langlebigkeit der Geräte, wichtige Teile wie Antrieb oder Elektronik vor eindringender Flüssigkeit zu schützen.

Das Problem wird folgend und beispielhaft anhand von Insulinpumpen erläutert. Das Problem tritt jedoch auch bei wiederverwendbaren Injektionspens und anderen Verabreichungsgeräten auf, weshalb die folgenden Erläuterungen keinesfalls einschränkend ausgelegt werden sollen.

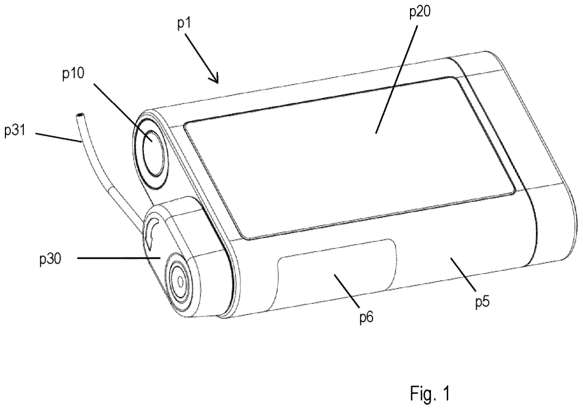

Aus dem Stand der Technik ist beispielsweise die Ypsopump der Anmelderin bekannt. Bei der Ypsopump handelt es sich um eine konventionelle Insulinpumpe, mit welcher Insulin aus einer Standard-Karpule verabreicht werden kann. Die Figuren 1 bis 3 zeigen die ganze Ypsopump, respektive Bereiche daraus.

Die Figuren 1 bis 3 zeigen vorbekannten Stand der Technik gemäss den Erläuterungen zum Hintergrund. Sie sind der EP 3110475 B1 entnommen, welche hiermit durch Referenz vollständig in den vorliegenden Dokumenten aufgenommen ist.

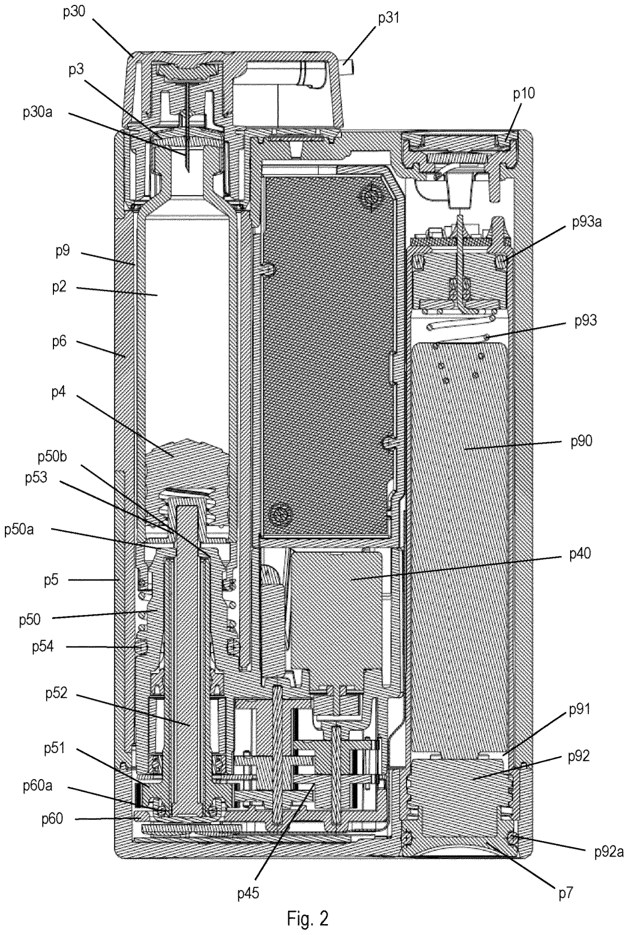

Figur 1 zeigt die Ypsopump p1 in einer perspektivischen Ansicht. Von aussen zu sehen ist das Gehäuse p5 mit dem Sichtfenster p6, durch welches der Zustand der Karpule p2 (siehe dazu Figur 2) kontrolliert werden kann. Weiter zu sehen ist Bedienknopf p10 und das Display p20, wobei es sich beim Display p20 um ein Touch-Display handelt. Weiter zu sehen sind der Infusionssetadapter p30 und der Infusionssetschlauch p31.

Figur 2 zeigt einen Schnitt durch die Ypsopump p1 und den Infusionssetadapter p30. Anhand Figur 2 kann die Funktionsweise der Ypsopump p1 kurz erläutert werden. Die Karpule p2 hat ein offenes Ende, welches durch den beweglich gelagerten Stopfen p4 verschlossen wird. An ihrem zweiten Ende ist die Karpule p2 mit einem Septum p3 verschlossen. Die Kanüle p30a des Infusionssetadapters p30 kann dieses Septum durchstechen, wodurch Medikament (Insulin) durch die Kanüle p30a in den Infusionssetschlauch p31 gelangen und in letzter Konsequenz subkutan dem oder der Patientin verabreicht werden kann. Der Stopfen p4 wird durch die Kolbenstange p52 bewegt. Figur 2 zeigt dabei eine volle Karpule p2 und entsprechend die Kolbenstange p52 vollständig zurückgezogen. Angetrieben wird die Kolbenstange p52 durch den Motor p40, welcher über das Getriebe p45 und die Antriebshülse 51 mit der Kolbenstange p52 verbunden ist. Die Kolbenstange p52 ist über die Verdrehsicherung p50a des Antriebsgehäuses p50 verdrehsicher aber axial verschiebbar mit diesem verbunden. Da das Antriebsgehäuse p50 fest im Gehäuse p5 angeordnet ist, ist somit auch die Kolbenstange p52 gegenüber dem Gehäuse p5 verdrehgesichert. Die Antriebshülse p51 weist auf ihrer Innenseite ein Gewinde auf p52b auf, welches in Gewindeverbindung mit dem proximalen Ende der Kolbenstange p52 ist. Wird die Antriebshülse p51 im ihre Achse rotiert, so wird die Kolbenstange p52 aufgrund der bestehenden Verdrehsicherung (siehe oben) nach proximal oder distal verschoben.

Figur 2 zeigt weiter das Batteriefach p91 mit der Batterie p90, dem negativen Anschluss p93 und dem positiven Anschluss p92. Der positive Anschluss p92 ist Teil des Batteriefachdeckels p7. Figur 2 zeigt weiter symbolisch die Elektronik p80.

Der Infusionssetadapter p30 hält die Karpule p2 in der Infusionspumpe p1 und ist über einen Bajonettverschluss in der Ypsopump p1 fixiert. Allerdings dichtet der Infusionssetadapter das Karpulenfach p9 nicht wasserdicht ab.

Um nun heikle Bereiche der Ypsopump p1, wie Elektronik p80 oder Motor p40 vor eintretenden Flüssigkeiten schützen zu können sind in der Ypsopump p1 verschiedene Dichtungselemente, insbesondere O-Ringe angeordnet. p54 und p60 schützen auf der Antriebsseite/Karpulenfach. P92a und p93 schützen auf der Seite des Batteriefachs. Das Gleitlager zwischen Kolbenstange p52 und der Verdrehsicherung p50a ist ebenfalls nicht flüssigkeitsdicht, so dass der Bereich zwischen Kolbenstange p52 und Drehhülse p51 kontaminiert werden könnte - Potential für weitere Verbesserungen.

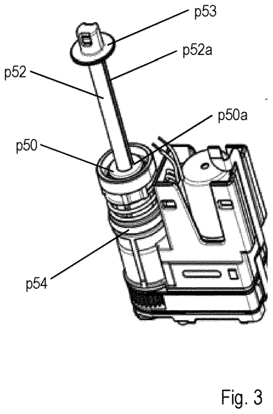

Wie in Figur 3 zu sehen, sind für die Verdrehsicherung der Kolbenstange p52 Längsnuten p52a angeordnet, in welche die Verdrehsicherung p50a (Nocken) eingreift. Diese Nut/Nocken Verdrehsicherung macht es schwierig in diesem Bereich, welcher auch als Lager funktioniert, ein elastisches Dichtelement zu positionieren, welches wirklich dichtet und gleichzeitig auch eine Schiebebewegung der Kolbenstange p52 innerhalb der Pumpe p1 zulässt.

Alternativ sind auch Kolbenstangen mit eckigem Querschnitt (Quadrat, Rechteck) bekannt, um eine Verdrehsicherung der Kolbenstange zu erzielen. Aber auch bei diesen klassischen Formen ergibt sich ein Dichtungsproblem, weil der Auflagedruck einer Dichtung, welche um den Querschnitt herum angeordnet ist, in den Ecken an Maximum erreicht und entlang der Kanten schwächer ist, so dass die Gefahr von Lecks erheblich ist.

Der Begriff "Produkt", "Medikament" oder "medizinische Substanz" umfasst im vorliegenden Zusammenhang jede fliessfähige medizinische Formulierung, welche geeignet ist zur kontrollierten Verabreichung mittels einer Kanüle oder Hohlnadel in subkutanes oder intramuskuläres Gewebe, beispielsweise eine Flüssigkeit, eine Lösung, ein Gel oder eine feine Suspension enthaltend einen oder mehrere medizinische Wirkstoffe. Ein Medikament kann also eine Zusammensetzung mit einem einzigen Wirkstoff oder eine vorgemischte oder co-formulierte Zusammensetzung mit mehreren Wirkstoffen aus einem einzelnen Behälter sein. Der Begriff umfasst insbesondere Arzneien wie Peptide (z.B. Insuline, Insulin enthaltende Medikamente, GLP 1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzyme und weitere Substanzen sowohl in fester (suspendierter) oder flüssiger Form. Der Begriff umfasst weiter auch Polysaccharide, Vakzine, DNS oder RNS oder Oligonukleotide, Antikörper oder Teile von Antikörpern sowie geeignete Basis-, Hilfs- und Trägerstoffe.

Der Begriff "distal" bezeichnet eine zum vorderen, einstechseitigen Ende der Verabreichungsvorrichtung beziehungsweise zur Spitze der Injektionsnadel hin gerichtete Seite oder Richtung. Demgegenüber bezeichnet die Angabe "proximal" eine zum hinteren, dem einstechseitigen Ende gegenüberliegenden Ende der Verabreichungsvorrichtung hin gerichtete Seite oder Richtung.

Die Begriffe "Verabreichungsgerät" und "Verabreichungsvorrichtung" werden in diesem Dokument synonym verwendet.

### DARSTELLUNG DER ERFINDUNG

Es ist eine Aufgabe der Erfindung, verbesserte Verabreichungsgeräte mit konventionellem Kolbstangenvortrieb bereitzustellen, bei welchen Bauteile vor Flüssigkeit geschützt werden müssen.

Die Aufgabe wird gelöst durch die Verabreichungsvorrichtung nach dem unabhängigen Anspruch. Vorteilhafte Ausführungsformen der Erfindung sind in den abhängigen Ansprüchen, der Beschreibung sowie den Figuren offenbart.

Ein Aspekt der Erfindung betrifft Verabreichungsvorrichtungen für fluide Medikamente gemäss der obenstehenden Definition für Medikament oder Produkt. Bei der Verabreichungsvorrichtung kann es sich um ein Injektionsgerät, zum Beispiel einen Injektionspen, oder ein Infusionsgerät handeln. Bei den Infusionsgeräten sind insbesondere Insulinpumpen und Pflasterpumpen (Patch Pumpen) mitgemeint, bei den Injektionsgeräten auch Autoinjektoren, Pens zum automatischen und mehrmaligen Abgeben von individuell einstellbaren Dosen (sog. Autopens) und Pflaster-Injektoren (patch injectors). Die Verabreichungsvorrichtungen können dabei monolithisch bis modular aufgebaut sein. Gemeinsam ist allen erfindungsgemässen Vorrichtungen, dass es Bereiche der Verabreichungsvorrichtung gibt, die vor Flüssigkeit geschützt werden sollen. Dabei kann es sich um mechanische, elektrische, elektronische, magnetische, elektromagnetische Bauteile, Baugruppen oder Kombinationen daraus handeln.

Eine erfindungsgemässe Verabreichungsvorrichtung umfasst ein Gehäuse, welches seinerseits aus mehreren Modulen bestehen kann, aber nicht muss. Die Module können für sich kleinere Gehäuse darstellen.

Im Gehäuse der Verabreichungsvorrichtung kann ein Reservoir zumindest teilweise im Innern des Gehäuses angeordnet werden. So kann zum Beispiel das distale Ende des Reservoirs, durch welches Medikament abgegeben wird, ausserhalb des Gehäuses lokalisiert sein. Das Reservoir umfasst ein inneres Volumen, welches zur Abgabe von Medikament verkleinerbar ist.

Beim Reservoir kann es sich um eine sogenannte Karpule im weiteren Sinne handeln, welche ein geschossenes Ende mit Septum umfasst, wobei das Septum mittels einer Kanüle durchstochen werden kann. Zum Beispiel kann es die Kanüle einer Injektionsnadel oder die Kanüle eines Infusionssetadapters sein. An ihrem anderen Ende ist die Karpule offen gestaltet, wobei ein beweglicher Stopfen das offene Ende verschliesst. So wird in der Karpule ein inneres Volumen gebildet. Dieses Volumen kann durch das Verschieben des Stopfens vergrössert oder verkleinert werden. Dem Fachmann sind verschiedene Materialisierungen der Karpule bekannt, aus Glas oder Kunststoff, mit rundem oder ovalem Querschnitt, resp. mit linearer Achse oder einer gebogenen Achse (z. B. toroidal).

Alternativ kann es sich beim Reservoir auch um einen Beutel handeln, welcher beim Verabreichen von Medikament ausgepresst wird.

Die erfindungsgemässe Verabreichungsvorrichtung umfasst weiter eine Antriebsvorrichtung. Die Antriebsvorrichtung ist zumindest teilweise im Gehäuse oder einem Modul des Gehäuses angeordnet. Die Antriebsvorrichtung dient dabei dazu, bei vorhandenem Reservoir, Medikament aus dem Reservoir auszutreiben. Die Antriebsvorrichtung umfasst einen Antrieb. Der Antrieb dient als Quelle für mechanische Energie. Beim Antrieb kann es sich um einen Motor, insbesondere einen Elektromotor, handeln. Alternativ und insbesondere wenn die Verabreichungsvorrichtung ein Injektionsgerät ist, kann es sich beim Antrieb um eine Anordnung von einer oder mehrerer Federn handeln. Die Aufgabe des Antriebes ist es, eine ebenfalls zumindest teilweise im Gehäuse oder teilweise in einem oder mehreren Modulen beweglich gelagerte Kolbenstange zu bewegen. Die Kolbenstange ist erfindungsgemäss verschiebbar aber nicht-rotierbar direkt oder indirekt im Gehäuse oder einem Modul gelagert. Der Antrieb kann direkt oder indirekt an die Kolbenstange gekoppelt sein. Insbesondere, wenn es sich beim Antrieb um einen Motor handelt, kann zwischen Antrieb und Motor ein Getriebe angeordnet sein, welches die Motorbewegung (üblicherweise eine rotierende, angetriebene Achse) in eine Verschiebebewegung der Kolbenstange umwandelt. Die Kopplung zwischen Antrieb (direkt oder indirekt) und Kolbenstange kann zum Beispiel über eine Gewindeverbindung zwischen Antrieb und Kolbenstange erfolgen. So kann zum Beispiel die Kolbenstange über ein Innengewinde verfügen, welches in Gewindeeingriff mit einer Gewindestange oder -spindel des Antriebs ist. Eine Rotation der Gewindestange oder -spindel evoziert aufgrund der Nicht-Rotierbarkeit der Kolbenstange eine Verschiebung der Kolbenstange. Die Gewindestange oder -spindel ist dabei vorteilhaft das Ausgangselement eines Getriebes zwischen einem Motor und der Kolbenstange.

Die Kolbenstange kann bei Ihrer Verschiebebewegung zum Beispiel einen Karpulenstopfen bewegen oder einen Beutel zusammenpressen.

Wie erwähnt ist die Kolbenstange zwar beweglich gelagert, Rotieren um die eigene Achse relativ zum Gehäuse oder dem Modul, in welchem die Kolbenstange lagert, ist jedoch nicht möglich. Erfindungsgemäss ist dazu ein Durchbruch in einer inneren Wandung des Gehäuses oder einer Wandung eines Moduls des Gehäuses vorhanden, an oder in welchem ein oder mehrere Verdrehsicherungselemente vorhanden sind. Die Kolbenstange ist durch diesen Durchbruch hindurch geführt und in diesem verschiebbar gelagert. Der Durchbruch kann dabei eine mehreckige Öffnung sein, durch welche die Kolbenstange hindurch geführt (Durchführung) wird, und wobei die mehreckige Form den Querschnitt der Kolbenstange in etwa abbildet. So kann analog zur obig beschriebenen Variante eine Verdrehsicherung erzielt werden. Alternativ kann die Verdrehsicherung über zusätzliche Elemente, welche fest am Durchbruch angeordnet sind erzielt werden. Auch kann die Wandung im Bereich des Durchbruchs zusätzlich mechanisch verstärkt sein, um zusätzlich auf die Wandung wirkende Kräfte aufnehmen zu können. Dies kann durch eine Erhöhung der Wanddicke geschehen, durch rippenartige Verstärkungen, oder weitere dem Fachmann bekannte Mittel.

Der Durchbruch ist weiter erfindungsgemäss mit einer Dichtung versehen, welche den Bereich zwischen dem Durchbruch und der Kolbenstange abdichtet, so dass, wenn die Kolbenstange durch den Durchbruch hindurchgeführt ist, keine Flüssigkeit von der einen Seite der Wandung auf die andere durch den Durchbruch hindurch gelangen kann. Die Dichtung ist aus einem zumindest elastisch deformierbaren Material gestaltet und sie kann den Umfang der Kolbenstange zumindest über einen Linienkontakt abdichten. Idealerweise ist der Kontakt zwischen Dichtung und Kolbenstange nicht nur linienförmig, sondern erstreckt sich auch entlang der Kolbenstangenachse, so dass eine Dichtungsfläche entsteht. Die Verschiebbarkeit der Kolbenstange bleibt dabei erhalten (vorteilhaft in beide Richtungen bei Mehrweggeräten).

Erfindungsgemäss weist die Kolbenstange zumindest über den axialen Bereich, welcher durch den Durchbruch verschoben wird, eine spezielle Querschnittform auf, welche eine verbesserte Dichtung ermöglicht. Die Form der Querschnitts entspricht dabei einem nichttrivialen Gleichdick (englisch: "orbiform curve"). Zur Veranschaulichung, was ein Gleichdick ist, wird hier die verständliche Definition von Wikipedia wiedergegeben (https://de.wikipedia.org/wiki/Gleichdick):
"Ein Gleichdick oder ein Bereich konstanter Breite ist anschaulich eine Figur, die überall gleich dick ist beziehungsweise die gleiche Breite besitzt. Den Rand einer solchen Figur bezeichnet man als Kurve konstanter Breite oder Orbiforme ("Kreisförmige").

Die Breite einer Kurve ist definiert als der Abstand zwischen zwei parallelen Geraden, die die Kurve auf gegenüberliegenden Seiten berühren. Diese Geraden nennt man Stützgeraden. Kurven konstanter Breite sind diejenigen Kurven, bei denen sich für den Abstand dieser Geraden immer derselbe Wert ergibt, unabhängig davon, an welcher Stelle der Figur die Geraden angreifen."

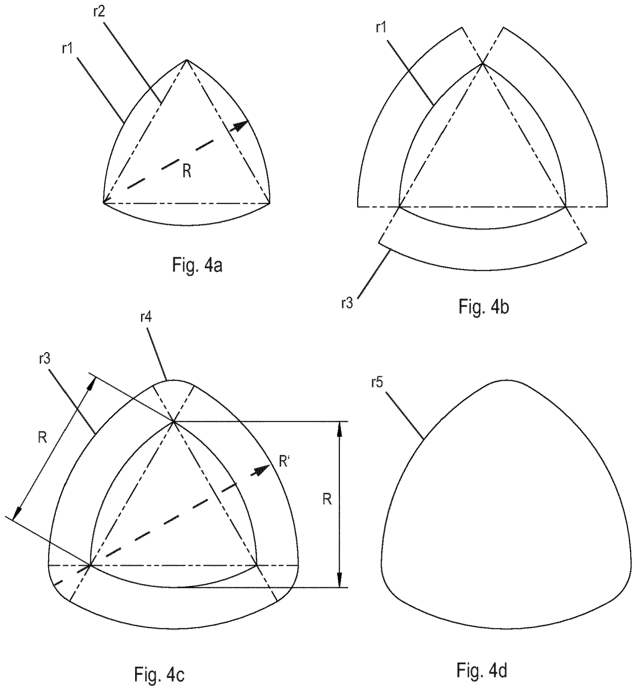

Das einfachste, notabene triviale Gleichdick ist der Kreis. Das einfachste nichttriviale Gleichdick, welches eine erfindungsgemässe Querschnittform darstellt, ist das sogenannte Reuleaux-Dreieck oder Kreisbogendreieck r1, wie es in Figur 4a gezeigt ist. Figur 4a zeigt weiter das gleichseitige Dreieck r2, welches der Konstruktion von Reuleaux-Dreieck r1 dient sowie den Radius R, welcher der Dicke des Gleichdicks und der Seitenlänge des Dreiecks entspricht. Figur 4d zeigt eine dreieckiges Gleichdick mit verrundeten Ecken r5, Figuren 4b und 4c zeigen die Konstruktion desselben. Die Ecken werden mit dem Radius r4 verrundet, wobei die Grösse des Radius |r4| dem zusätzlichen Auftrag am gesamten Gleichdick entspricht, so dass die resultierende Dicke des Gleichdicks als R' = R + 2*|r4| berechnet werden kann. r3 entspricht dem Radius R plus |r4|, also nur einmal |r4|. Hierbei kann Grösse des Radius r3 beliebige Grössen|r3| annehmen, wobei |r3| > R, wodurch sich |r4| aus |r3| -R ergibt.

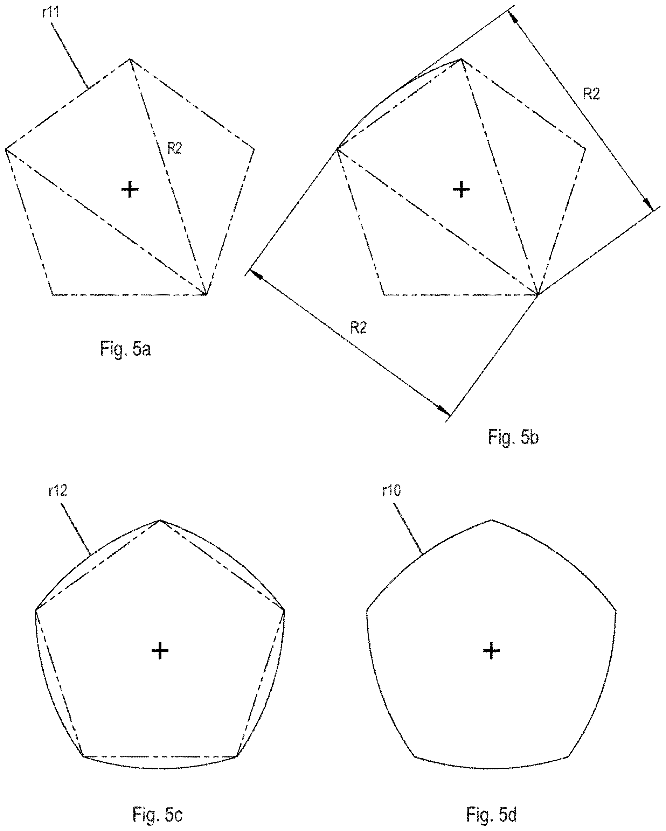

Zwischen Reuleaux-Dreieck und Kreis gibt es unendlich viele weitere Gleichdicke, gemein ist ihnen mit dem Reuleaux-Dreieck die ungerade Zahl an Ecken und die konvexe Form zwischen den Ecken. Als weiteres Beispiel sind das fünfeckige Gleichdick und dessen Konstruktion in den Figuren 5a bis 5d gezeigt, wobei 5d das fertige Gleichdick r10 zeigt. Figur 5a das Fünfeck r11, welches als Ausgangspunkt für die Konstruktion des fünfeckigen Gleichdicks dient. R2 ist dabei der Radius für die Kreisbögen r12 und entspricht der Dicke des Gleichdicks r10.

Gleichdicke müssen auch nicht gleichseitig sein wie das Reuleaux-Dreieck. Bei der Konstruktion müssen einfach gewisse Regeln eingehalten werden, wie sie unter
https://web.archive.org/web/20200223001951/http://www.mathematische-basteleien.de/gleichdick.htm
abgerufen werden können (archiviert am 23.02.2020 bei archive.org). Die konvex gebogenen Seiten sind zentral bei der Dichtung des Durchbruchs, wodurch regelmässigere Druckverteilungen in der Dichtung (resp. Flächenpressung, die auf die Kolbenstange wirkt) erzielt werden können. Trotzdem erlauben es solche Querschnittformen eine Verdrehsicherung zu erzielen.
in einem Aspekt der Erfindung ist der Bereich der Wandung, welche den Durchbruch aufweist, als 2-Komponenten-Spritzgussteil ausgeführt. Wobei der tragende Teil, die eigentliche Wandung aus einem ersten Material gespritzt wird (erste Komponente, technisch auch Vorspritzling genannt) und der Teil der Dichtung aus einem zweiten Material, welches zumindest elastisch deformierbar ist, gespritzt wird (zweite Komponente). Einerseits ermöglicht dieses Vorgehen das Erstellen des Bereichs der Wandung in einem Arbeitsgang mit passender Dichtung. Andererseits kann der Bereich der Wandung noch weitere Durchbrüche enthalten, welche ebenfalls im gleichen Arbeitsgang abgedichtet werden können. Dabei kann es sich vorteilhaft um Dichtungen für Bedienelemente (Knöpfe) oder Dichtungen für elektrische Durchführungen handeln.

In einer Ausgestaltung des Aspekts handelt es sich beim Material für die zweite Komponente um ein spritzgussfähiges, insbesondere thermoplastisches, Polyurethan oder Polyamid. Alternativ kann es sich verallgemeinert auch um ein thermoplastisches Elastomer handeln. Bei einer weiteren Alternative besteht die zweite Komponente aus Silikon, zum Beispiel einem Silikon aus zwei Bestandteilen, welches in der Spritzgussvorrichtung aushärtet.

In einem Aspekt der Erfindung handelt es sich bei der Verabreichungsvorrichtung um eine Infusionspumpe im Stile der obig beschriebenen Ypsopump. Alternativ kann es sich bei der Infusionspumpe um eine modulare Pumpe handeln, welche zum Beispiel aus einem wiederverwendbaren Modul mit Elektronik und Antrieb sowie einem Einwegmodul bestehen. Im Einwegmodul können so dann zum Beispiel das Medikamentenreservoir und vorteilhaft eine Energiequelle angeordnet sein. In einer weiteren Alternative kann es sich bei der Infusionspumpe um eine sogenannte Pflaster- oder Patch-Pumpe handeln, welche auf die Haut der benützenden Person geklebt wird, insbesondere kann es sich dabei um eine modulare Patch-Pumpe handeln.

In einem Aspekt der Erfindung handelt es sich bei der Verabreichungsvorrichtung um ein Injektionsgerät. Das Injektionsgerät kann insbesondere Stift- oder Pen-förmig sein. Alternativ kann es sich beim Injektionsgerät um einen sogenannten Patch-Injektor handeln, welcher für eine einmalige Injektion von Medikament auf die Haut der benützenden Person geklebt werden kann. In einer weiteren Alternative kann es sich beim Injektionsgerät um einen stiftförmigen Autoinjektoren handeln, wie er zum Beispiel von der Anmelderin als Ypsomate auf dem Markt ist. In einer noch weiteren Alternative handelt es sich beim Injektionsgerät um einen Injektionspen, mit welchem mehrere Dosen automatisch ausgeschüttet werden können, wie zum Beispiel dem bekannten ServoPen der Anmelderin. Die Injektionsgeräte können beispielhaft Elektronik enthalten, welche geschützt werden muss.

### FIGUREN

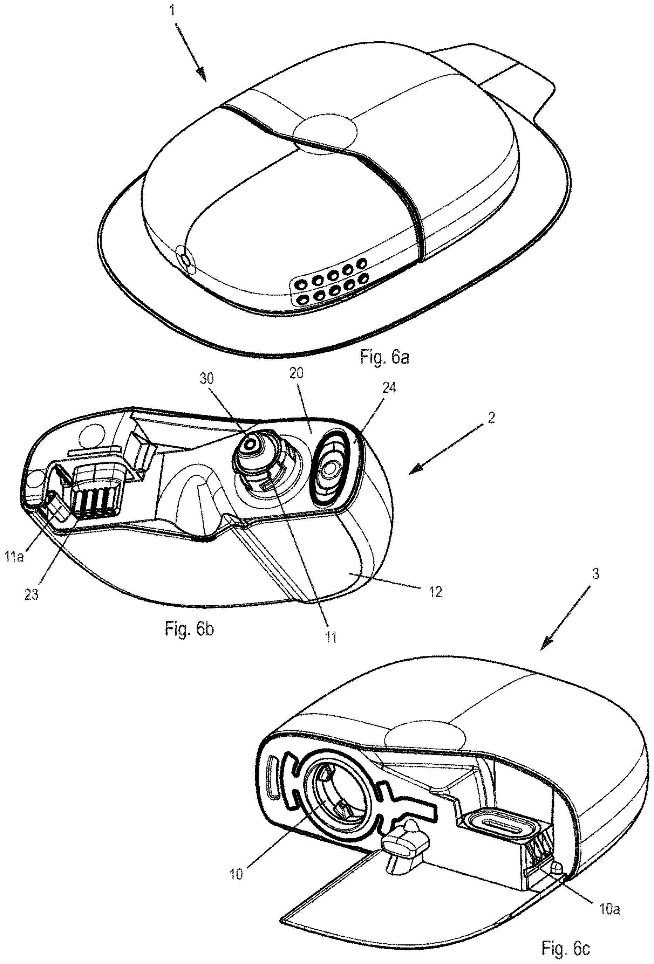

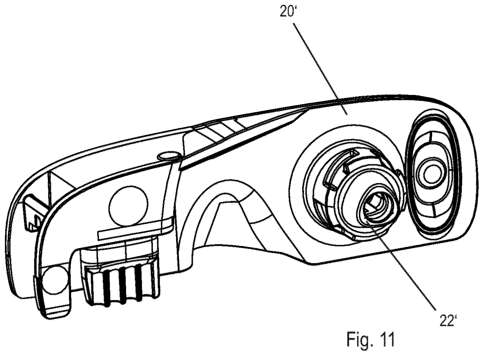

Die Figuren 1 bis 3 zeigen vorbekannten Stand der Technik gemäss den Erläuterungen zum Hintergrund. Figuren 4 und 5 zeigen Details zu den verschiedenen Formen des Gleichdicks. Die Figuren 6 bis 10 zeigen eine erfindungsgemässe Ausführungsform. Figur 11 zeigt eine alternative Ausgestaltung.

In Zusammenhang mit den weiteren, angehängten Figuren werden nachfolgend bevorzugte Ausführungsarten der Erfindung beschrieben. Diese sollen grundsätzliche Möglichkeiten der Erfindung aufzeigen und keinesfalls einschränkend ausgelegt werden.
- Fig. 1: Stand der Technik: Ypsopump
- Fig. 2: Stand der Technik: Längsschnitt durch Ypsopump mit Dichtungen
- Fig. 3: Stand der Technik: Antriebseinheit mit Kolbenstange und Dichtung
- Fig. 4a-d: Reuleaux-Dreieck und verrundetes Reuleaux-Dreieck
- Fig. 5a-d: Fünfeckiges Gleichdick und Konstruktion desselben
- Fig. 6a: Erfindungsgemässe Infusionspumpe (modulare Infusionspumpe), perspektivische Ansicht
- Fig. 6b: Pumpenmodul der erfindungsgemässen Infusionspumpe
- Fig. 6c: Reservoirmodul der erfindungsgemässen Infusionspumpe, wobei das Pflaster resp. der adhäsive Patch nicht gezeigt ist
- Fig. 7a: Pumpenmodul der erfindungsgemässen Infusionspumpe mit ausgefahrener Kolbenstange, wobei Kolbenstange Querschnitt einem Gleichdick mit verrundeten Ecken entspricht
- Fig. 7b: Stirnelement des Pumpenmoduls, Aussenseite des Stirnelements
- Fig. 7c: Stirnelement des Pumpenmoduls, Innenseite des Stirnelements
- Fig. 8a: Stirnelement des Pumpenmoduls mit eingesetzter Kolbenstange (zurückgezogene Position)
- Fig. 8b: Vertikalschnitt durch das Stirnelement und die eingesetzte Kolbenstange im Zustand von Fig. 8a
- Fig. 9a: Stirnelement des Pumpenmoduls mit eingesetzter Kolbenstange (ausgeschobene Position)
- Fig. 9b: Vertikalschnitt durch das Stirnelement und die eingesetzte Kolbenstange im Zustand von Fig. 9a
- Fig. 10a: Stirnelement des Pumpenmoduls mit eingesetzter Kolbenstange: 2-Komponenten-Spritzgussteil.
- Fig. 10b: 2-Komponenten-Spritzgussteil nur harte Komponente (Vorspritzling)
- Fig. 10c: 2-Komponenten-Spritzgussteil nur Dichtungskomponente
- Fig. 10d: Vertikalschnitt durch Stirnelement aus Fig. 10a im Bereich des Resetknopfs
- Fig. 10e: Vertikalschnitt durch Stirnelement aus Fig. 10a im Bereich der elektrischen Steckverbindung
- Fig. 11: Alternative Ausführungsform des Stirnelements

### FIGURENBESCHREIBUNG

Figur 6a zeigt eine erfindungsgemässe Verabreichungsvorrichtung in Form der Pflaster- oder Patchpumpe 1. Die Patchpumpe 1 ist sehr ähnlich aufgebaut, wie in der europäischen Patentanmeldung EP20181599.0 beschrieben, welche hiermit durch Verweis vollständig in das vorliegende Dokument übernommen ist. Details zur grundsätzlichen Technologie der Patchpumpe 1 können der Anmeldung EP20181599.0 direkt und eindeutig entnommen werden.

Patchpumpe 1 ist, wie in den Figuren 6b und 6c gezeigt, modular aufgebaut und umfasst ein Pumpenmodul 2 und ein Reservoirmodul 3, welche über den Bajonettverschluss mit den Teilen 10 am Reservoirmodul 3 und 11 am Pumpenmodul lösbar miteinander verbunden werden können. Sind Reservoirmodul 3 und Pumpenmodul 2 über den Bajonettverschluss mit einander verbunden, so greift die Rastfeder 11a des Pumpenmoduls 2 hinter die Rastnase 10a des Reservoirmoduls 2, so dass sich die beiden Module nicht ungewollt voneinander lösen können. Das Pumpenmodul 2 ist wiederverwendbar und umfasst die Pumpenelektronik (nicht gezeigt) und den Antrieb (nicht gezeigt, mit Motor, Getriebe, Übertragungselementen) sowie der beweglich im Pumpenmodul 2 gelagerten Kolbenstange 30. Weiter kann das Pumpenmodul 2 eine Energiequelle in Form einer mehrfach wiederaufladbaren Batterie umfassen.

Das Reservoirmodul 3 umfasst das Reservoir, eine Energiequelle in Form von zum Beispiel einer Batterie sowie einer Infusionsleitung, welche das zu verabreichende Medikament vom Reservoir ins Gewebe leiten kann. Wenn das Reservoirmodul 3 und das Pumpenmodul 2 zusammengebaut sind kann die erwähnte Batterie dazu dienen die wiederaufladbare Batterie (oder einen analogen Energiespeicher, z. B. eine Kapazität) zu laden. Das Reservoir (nicht gezeigt) hat typischerweise in etwa die Form einer Karpule, wobei ein beweglicher Stopfen (nicht gezeigt) in Reservoir gelagert ist. Durch ein Verschieben des Stopfens im Reservoir lässt sich das Volumen im Reservoir verkleinern oder vergrössern. Wenn Pumpenmodul 2 und Reservoirmodul 3 zusammengebaut sind, lässt sich der Stopfen im Reservoir durch eine axiale Bewegung der Kolbenstange verschieben. Typisch wäre in diesem Fall eine Bewegung der Kolbenstange 30 in das Reservoirmodul 3 hinein und folgend einer Verkleinerung des Volumens im Reservoir und schlussendlich einer Ausschüttung von Medikament durch die Infusionsleitung ins Gewebe der benutzenden Person.

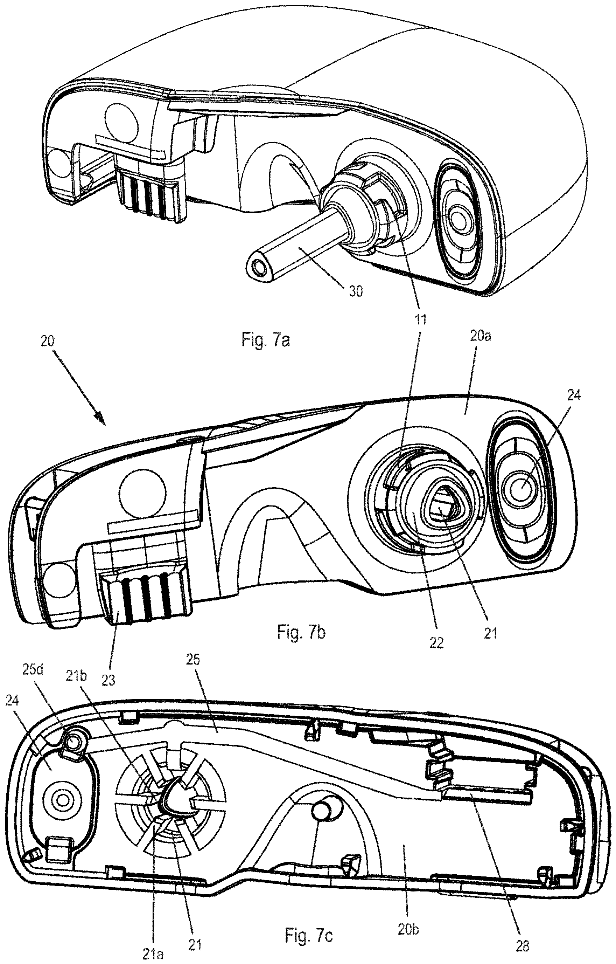

Wie beschrieben, kann die Kolbenstange 30 bewegt werden, insbesondere teilweise aus dem Pumpenmodul 2 heraus bewegt werden. Das Pumpenmodul 2 umfasst ein Gehäuse 12 und ein Stirnelement 20. Am Stirnelement 20 sind, wie zum Beispiel in den Figuren 6b und 7a zu erkennen, verschiedene Elemente angeordnet. Es sind dies der Bajonettverschluss 11 (Komponente Pumpenmodul, die um den Durchbruch 21 herum angeordnet ist, stutzenförmig), der Durchbruch 21 für die Kolbenstange 30, der Stecker für die elektrische Steckverbindung 23 sowie der Resetknopf 24 - andere oder weitere Elemente wären möglich. Das Stirnelement weist eine Vorderseite 20a und eine Rückseite 20b auf, siehe Figuren 7b und 7c.

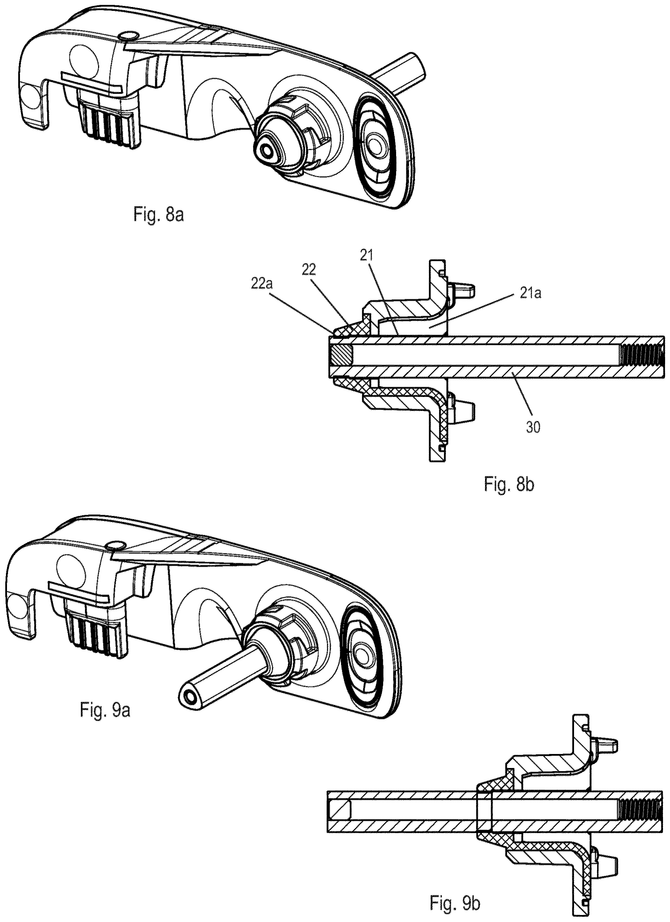

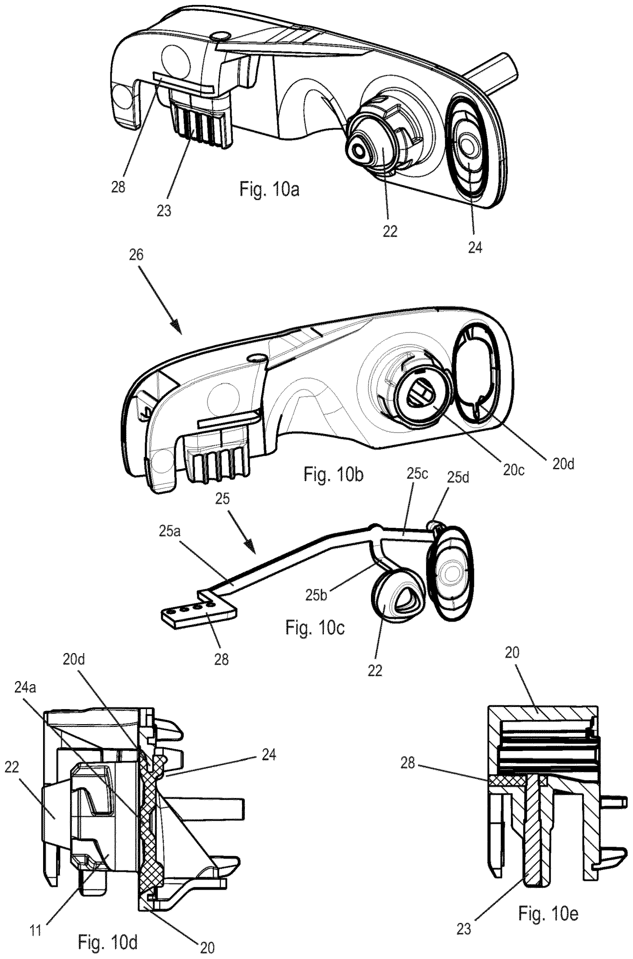

Die Kolbenstange 30 ist im Durchbruch 21 geführt, sie Figuren 7a bis 7c. Die Kolbenstange 30 weist erfindungsgemäss einen Querschnitt auf, welcher der Form eines dreieckigen Gleichdicks mit verrundeten Ecken entspricht. Der Durchbruch 21 hat eine Form, die in etwa dem Negativ des Gleichdicks entspricht, so dass die Kolbenstange 30 durch den Durchbruch 21 hindurch verschoben werden kann, jedoch nicht um die Achse der Kolbenstange 30 rotiert werden kann. Die Kolbenstange 30 ist also beweglich aber verdrehgesichert im Durchbruch 21 gelagert. Der Durchbruch 21 wird dabei durch die Rippen 21a verstärkt, wobei die Rippen 21a mit ihren Stirnflächen 21b die Kolbenstange 30 insbesondere auch führen können (siehe dazu insbesondere die Figuren 8a bis 9b). Um das Innere des Pumpenmoduls vor Flüssigkeiten zu schützen auf der Vorderseite 20a des Stirnelements 20 die Dichtung 22 angeordnet. Die Dichtung 22 empfindet dabei die Form des Gleichdicks des Querschnitts der Kolbenstange 30 nach. Die Öffnung der Dichtung 22 ist im undeformierten Zustand kleiner als der Querschnitt der Kolbenstange 30. Wird die Kolbenstange 30 durch die Öffnung der Dichtung 22 geschoben, so wird diese Öffnung aufgedehnt. Deshalb ist die Dichtung aus einem deformierbaren Werkstoff hergestellt, insbesondere einem Elastomer. Die Dichtung kann somit verhindern, dass Flüssigkeit die Grenzfläche zwischen Kolbenstange 30 und Dichtung 22 (und somit Durchbruch 21) durchdringen kann. Die Dichtung 22 könnte an sich auf den Stutzen des Bajonettverschlusses 11 geklebt sein. In der vorliegenden Ausführungsform handelt es sich beim Stirnelement 20 jedoch um ein 2-Komponenten-Spritzgussteil, welches es erlaubt, Wand des Stirnelements 20 und die Dichtung 22 mit einem dichten Haftverbund als ein Teil herzustellen. Wie in den Figuren 10b und 10c dargestellt, besteht das Stirnelement 20 aus einer Komponente 26, genannt Stirnelementplatte 26, und dem Dichtungsensemble 25. Für das bessere Verständnis des Stirnelements 20 wurde das Stirnelement 20 in den Figuren 10b und 10c in seine Komponenten aufgeteilt. Das Dichtungsensemble ist dabei aus einem weicheren Material (insbesondere elastomeren Material) gefertigt als die Stirnelementplatte 26, welche unter anderem die Kolbenstange 30 führen muss. Die Stirnelementplatte 26 muss dazu eine gewisse Steifigkeit und auch Festigkeit aufweisen. Bei der Herstellung wird zuerst die Stirnelementplatte gespritzt und dann in derselben Spritzgussform das Dichtungsensemble 25.

Wie in Figur 10c zu sehen umfasst das Dichtungsensemble 25 nicht nur die Dichtung 22, sondern auch die Dichtung 24 für den Resetknopf. Der eigentliche Resetschalter ist in den vorliegenden Zeichnungen nicht gezeigt, er dient dazu Einstellungen in der Pumpenelektronik des Pumpenmoduls 2 zurückzusetzen, den entsprechenden Speicher zu löschen und/oder die Elektronik des Pumpenmoduls 2 neu zu starten (zum Beispiel durch einen kurzen Stromunterbruch). Figur 10d zeigt einen Vertikalschnitt durch das Stirnelement 20 im Bereich des Resetknopfs, speziell der Dichtung 24. Die Dichtung ist eine strukturierte Membran, wobei das Bedienelement 24a der Dichtung 24 gegenüber der umliegenden Wand des Stirnelements 20 in Richtung der Vorderseite 20a nicht hervorsteht, sondern plan oder versenkt liegt, um eine unbeabsichtigte Betätigung des Resetknopfs zu verhindern. Um die Dichtung 24 fest im Stirnelement 20 halten zu können (resp. die Haftung zu verbessern), sind am Übergang zwischen Dichtung 24 und dem Stirnelement 20 Halteelemente 20d (siehe Figuren 10b und 10d) am Stirnelement angeordnet (ein chemischer Haftverbund besteht vorteilhaft schon). Beim Angiessen der Dichtung 24 an die Stirnelementplatte 26 entsteht dadurch auch eine Verzahnung zwischen Stirnelement 20 und der Dichtung 24.

Weiter umfasst das Dichtungsensemble 26 weiter die Dichtung 28 für die elektrischen Kontakte des elektrischen Steckverbinders 23. In der gezeigten Ausführung weist die Dichtung 28 gegenüber den als Stiften ausgeführten Kontakten ein geometrisches Untermass auf, wodurch eine radiale Anpressung der Dichtung auf den Stiften erzeugt wird. Sie soll verhindern, dass Flüssigkeit entlang der elektrischen Kontakte ins Innere des Pumpenmoduls 2 dringen kann. Figur 10e zeigt einen Vertikalschnitt durch das Stirnelement 20 im Bereich des Steckverbinders 23, wobei rechts in der Figur die Vorderseite des Stirnelements 20 ist.

Damit bei der Herstellung des Stirnelement nicht alle beschriebenen Dichtung separat angespritzt werden müssen, sind die Dichtungen untereinander über die Arme 25a, 25b und 25b miteinander verbunden. An der Stirnelementelementplattenrückseite (26) sind dazu entsprechende Kanäle vorgesehen. Angespritzt werden in dieser Ausführungsform all Dichtungen über den Öppel 25d. Auf diese Art und Weise lässt sich die Stirnplatte 20 mit Dichtungen elegant in einem Spritzgussvorgang herstellen.

Figur 11 zeigt eine alternative Ausgestaltung des Stirnelements 20, nämlich das Stirnelement 20' mit einer abgewandelten Dichtung 22`. Bei dieser Dichtung 22` ist die Öffnung kreisförmig und nicht dem Gleichdick der Kolbenstange 30 nachempfunden. Eine funktionierende Dichtungsfunktion der Dichtung 22 ist trotzdem möglich, weil eben genau die Form des Gleichdicks eine homogenere Spannungsverteilung in der Dichtung zulässt, so dass zum Beispiel im Bereich der Ecken relativ zu den Kanten keine übermässige Spannungsdifferenz entsteht, wie sie bei einem zum Beispiel gleichseitigen Dreieck als Querschnitt für die Kolbenstange entstehen würde. Trotzdem erlaubt die Form des Gleichdicks, wie beschrieben eine sichere Verdrehsicherung.

### BEZUGSZEICHENLISTE

### Stand der Technik:

- p1: Ypsopump
- p2: Karpule
- p3: Septum
- p4: Stopfen
- p5: Gehäuse
- p6: Sichtfenster
- p7: Batteriedeckel
- p9: Karpulenfach
- p10: (Bedien-)Knopf
- p20: Touch-Display
- p30: Infusionssetadapter
- p30a: Kanüle
- p31: Infusionsleitung
- p40: Motor
- p45: Getriebe

- p50: Antriebsgehäuse
- p50a: Verdrehsicherung
- p51: Antriebshülse
- p52: Kolbenstange
- p52a: Führungsnut
- p53: Flansch
- p54: Dichtung (O-Ring)
- p60: Lagerplatte
- p60a: Dichtung
- p90: Batterie
- p91: Batteriefach
- p92: positiver Batteriekontakt / Anschluss
- p92a: Dichtung
- p93: negativer Batteriekontakt / Anschluss
- p93a: Dichtung

### Gleichdick-Figuren:

- r1: Reuleaux-Dreieck (einfachstes nichttriviales Gleichdick)
- R: Dicke
- r2: Gleichseitiges Dreieck, als Basis für die Konstruktion des Reuleaux-Dreiecks
- r3: Erweiterter Bogen für Konstruktion Gleichdick mit verrundeten Ecken
- r4: Eckradius (|r4| entspricht der Grösse des Radius)
- R': Dicke (R` = R + 2*|r4|)
- r5: Gleichdick mit verrundeten Ecken
- r10: Gleichdick mit fünf Ecken
- r11: Konstruktionsfünfeck
- r12: Kreisbogen des fünfeckigen Gleichdicks
- R2: Dicke

### Verabreichungsvorrichtungen:

- 1: Verabreichungsvorrichtung in Form einer modularen Patch Pumpe
- 2: (wiederverwendbares) Pumpen Modul
- 3: Reservoirmodul
- 10: Bajonettverschluss (Teil Reservoirmodul 3)
- 10a: Rastnase
- 11: Bajonettverschluss (Teil Pumpenmodul 2)
- 11a: Rastfeder
- 12: Gehäuse Pumpmodul
- 20: Stirnelement des Pumpenmoduls 2, ausgeführt als 2k-Spritzgussteil
- 20`: Alternatives Stirnelement
- 20a: Vorderseite Stirnelement 20
- 20b: Rückseite Stirnelement 20
- 20c: Stirnfläche
- 20d: Halteelemente
- 21: Durchbruch
- 21a: Rippen
- 21b: Stirnflächen
- 22: Dichtung Kolbenstange
- 22`: Alternative Dichtung Kolbenstange
- 22a: Dichtfläche
- 23: elektrisches Steckverbindungselement mit elektrischen Kontakten
- 24: Dichtung Resetknopf (eigentlicher Resetknopf nicht gezeigt)
- 24a: Bedienelement
- 25: Dichtungsensemble (Dichtungskomponente des Stirnelements 20)
- 25a: Dichtungsverbindung zu Dicht 28 für die elektrischen Kontakte
- 25b: Dichtungsverbindung zu Dichtung 22 für die Kolbenstange 30
- 25c: Dichtungsverbindung zu Dichtung 24 für den Resetknopf
- 25d: Öppel
- 26: Stirnelementplatte (tragende Komponente des Stirnelements 20
- 28: Dichtung für elektrische Kontakte 23
- 30: Kolbenstange mit der Querschnittform eines Gleichdicks

## Patentansprüche

1. Verabreichungsvorrichtung (1) zum Verabreichen eines fluiden Medikamentes zumindest umfassend
a. ein Gehäuse, welches insbesondere modular ausgebildet sein kann, und wobei die Module (2, 3) des Gehäuses lösbar miteinander verbindbar sind,
b. ein Reservoir mit einem variablen Innenvolumen zum Speichern des fluiden Medikamentes, wobei das Reservoir zumindest teilweise im Gehäuse, insbesondere in einem Reservoirmodul (3) des Gehäuses, angeordnet ist,
c. eine Antriebsvorrichtung, wobei die Antriebsvorrichtung zumindest teilweise im Gehäuse (12), insbesondere in einem Antriebsmodul (2) des Gehäuses, angeordnet ist, zumindest umfassend
i. einen Antrieb, insbesondere einen Motor oder eine Feder,
ii. eine beweglich im Gehäuse gelagerte Kolbenstange (30), welche durch den Antrieb bewegbar ist und wobei durch die Bewegung das variable Innenvolumen änderbar ist,
wobei die Kolbenstange durch eine innere Wandung des Gehäuses und/oder eine äussere Wandung eines Moduls des Gehäuses durch einen Durchbruch (21) hindurchführbar ist
wobei die Kolbenstange verschiebbar aber nicht rotierbar im Durchbruch gelagert ist, wobei das Lager zwischen Kolbenstange und Durchbruch eine elastisch deformierbare Dichtung (22) umfasst, welche verhindert, dass Flüssigkeit den Durchbruch passieren kann,
jedoch ein Verschieben der Kolbenstange weiter zulässt,
**dadurch gekennzeichnet, dass**
der Form des Umfangs des Querschnitts der Kolbenstange in etwa ein nichttriviales Gleichdick darstellt.

2. Eine Verabreichungsvorrichtung nach dem vorhergehenden Anspruch, wobei es sich beim Gleichdick um ein Gleichdick mit drei oder mehr Ecken handelt, speziell um ein Gleichdick mit 5 oder 7 Ecken.

3. Eine Verabreichungsvorrichtung nach Anspruch 1, wobei es sich beim Gleichdick um ein Gleichdick mit verrundeten Ecken handelt.

4. Eine Verabreichungsvorrichtung nach einem der vorhergehenden Ansprüche, wobei das Gehäuse modular ist und aus einem Reservoirmodulgehäuse und einem Antriebsmodulgehäuse besteht, welche lösbar miteinander verbindbar sind, wobei das Antriebsmodulgehäuse aus Kunststoff gefertigt und der Bereich der Wandung mit dem Durchbruch ein 2-Komponenten-Spritzugussteil ist, wobei das Material der ersten Komponente für die Wandung verwendet wird und das Material der zweite Komponente das Material für die Dichtung ist.

5. Eine Verabreichungsvorrichtung nach Anspruch 4, wobei das Material der zweiten Komponente ein thermoplastisches Elastomer (TPE) ist, insbesondere ein thermoplastisches Polyurethan oder ein thermoplastisches Polyamid.

6. Eine Verabreichungsvorrichtung nach Anspruch 4, wobei das Material der zweiten Komponente ein Silikon ist.

7. Eine Verabreichungsvorrichtung nach Anspruch 4, 5 oder 6, wobei der Bereich der Wandung mit dem Durchbruch mindestens einen weiteren Durchbruch aufweist, welcher eine mindestens eine weitere Dichtung umfasst, wobei diese mindestens weitere Dichtung ebenfalls Teil des 2-Kompenenten-Spritzgussteils ist.

8. Eine Verabreichungsvorrichtung nach Anspruch 6, wobei es sich beim mindestens einen weiteren Durchbruch um eine Durchführung für ein Bedienelement und/oder einen oder mehrere elektrische Kontakte handelt.

9. Eine Verabreichungsvorrichtung nach einem der vorhergehenden Ansprüche, **dadurch gekennzeichnet, dass** die Verabreichungsvorrichtung eine Infusionspumpe ist.

10. Eine Verabreichungsvorrichtung nach Anspruch 8, **dadurch gekennzeichnet, dass** es sich bei der Infusionspumpe um eine Pflasterpumpe oder eine Patch Pumpe handelt.

11. Eine Verabreichungsvorrichtung nach einem der Ansprüche 1 bis 7, **dadurch gekennzeichnet, dass** die Verabreichungsvorrichtung ein Injektionsgerät ist, insbesondere ein stiftförmiges Inj ektionsgerät.

## Claims

1. Administration device (1) for administering a fluid medicament, at least comprising
a. a housing, which can in particular be of modular design, and the modules (2, 3) of the housing being detachably connectable to one another,
b. a reservoir having a variable internal volume for storing the fluid medicament, the reservoir being arranged at least partially in the housing, in particular in a reservoir module (3) of the housing,
c. a drive device, the drive device being arranged at least partially in the housing (12), in particular in a drive module (2) of the housing, at least comprising
i. a drive, in particular a motor or a spring,
ii. a piston rod (30) movably mounted in the housing, which is movable by means of the drive, and the variable internal volume being changeable by means of the movement,
the piston rod being guidable through an inner wall of the housing and/or an outer wall of a module of the housing through an aperture (21),
the piston rod being mounted in the aperture so that it can move but not rotate,
the bearing between the piston rod and the aperture comprising an elastically deformable seal (22) which prevents liquid from passing through the aperture but still allows the piston rod to move,
**characterized in that**
the shape of the circumference of the cross-section of the piston rod represents substantially a non-trivial orbiform curve.

2. Administration device according to the preceding claim, wherein the orbiform curve is an orbiform curve having three or more corners, especially an orbiform curve having 5 or 7 corners.

3. Administration device according to claim 1, wherein the orbiform curve is an orbiform curve having rounded corners.

4. Administration device according to any of the preceding claims, wherein the housing is modular and consists of a reservoir module housing and a drive module housing which can be detachably connected to one another, wherein the drive module housing is made of plastic and the region of the wall having the aperture is a 2-component injection-molded part, wherein the material of the first component is used for the wall and the material of the second component is the material for the seal.

5. Administration device according to claim 4, wherein the material of the second component is a thermoplastic elastomer (TPE), in particular a thermoplastic polyurethane or a thermoplastic polyamide.

6. Administration device according to claim 4, wherein the material of the second component is a silicone.

7. Administration device according to claim 4, 5 or 6, wherein the region of the wall having the aperture has at least one further aperture which comprises at least one further seal, wherein said at least one further seal is also part of the 2-component injection-molded part.

8. Administration device according to claim 6, wherein the at least one further aperture is a feedthrough for an operating element and/or one or more electrical contacts.

9. Administration device according to any of the preceding claims, **characterized in that** the administration device is an infusion pump.

10. Administration device according to claim 8, **characterized in that** the infusion pump is a plaster pump or a patch pump.

11. Administration device according to any of claims 1 to 7,
**characterized in that** the administration device is an injection device, in particular a pen-shaped injection device.

## Revendications

1. Dispositif d'administration (1) permettant d'administrer un médicament fluide, comprenant au moins
a. un boîtier qui peut être réalisé en particulier de manière modulaire, et dans lequel les modules (2, 3) du boîtier peuvent être reliés entre eux de manière amovible,
b. un réservoir comportant un volume intérieur variable pour le stockage du médicament fluide, dans lequel le réservoir est disposé au moins partiellement dans le boîtier, en particulier dans un module pour réservoir (3) du boîtier,
c. un dispositif d'entraînement, dans lequel le dispositif d'entraînement est disposé au moins partiellement dans le boîtier (12), en particulier dans un module d'entraînement (2) du boîtier, comprenant au moins
i. un entraînement, en particulier un moteur ou un ressort,
ii. une tige de piston (30) montée de manière à pouvoir se déplacer dans le boîtier, laquelle peut être déplacée par l'entraînement, et dans lequel le volume intérieur variable peut être modifié par le déplacement,
dans lequel la tige de piston peut être passée à travers une paroi intérieure du boîtier et/ou une paroi extérieure d'un module du boîtier par un passage (21),
dans lequel la tige de piston est montée de manière coulissante mais non rotative dans le passage,
dans lequel le palier entre la tige de piston et le passage comprend un joint (22) élastiquement déformable qui empêche le liquide de passer à travers le passage, mais qui permet à la tige de piston de continuer à coulisser,
**caractérisé en ce que**
la forme de la circonférence de la section transversale de la tige de piston représente approximativement une courbe de largeur constante non triviale.

2. Dispositif d'administration selon la revendication précédente, dans lequel la courbe de largeur constante est une courbe de largeur constante comportant trois angles ou plus, de manière particulière une courbe de largeur constante comportant 5 ou 7 angles.

3. Dispositif d'administration selon la revendication 1, dans lequel la courbe de largeur constante est une courbe de largeur constante comportant des angles arrondis.

4. Dispositif d'administration selon l'une des revendications précédentes, dans lequel le boîtier est modulaire et se compose d'un boîtier de module pour réservoir et d'un boîtier de module d'entraînement qui peuvent être reliés entre eux de manière amovible, dans lequel le boîtier de module d'entraînement est fabriqué en matière plastique et la zone de la paroi comportant le passage est une pièce moulée par injection à deux composants, dans lequel le matériau du premier composant est utilisé pour la paroi et le matériau du second composant est le matériau pour le joint.

5. Dispositif d'administration selon la revendication 4, dans lequel le matériau du second composant est un élastomère thermoplastique (TPE), en particulier est un polyuréthane thermoplastique ou un polyamide thermoplastique.

6. Dispositif d'administration selon la revendication 4, dans lequel le matériau du second composant est une silicone.

7. Dispositif d'administration selon la revendication 4, 5 ou 6, dans lequel la zone de la paroi comportant le passage présente au moins un autre passage qui comprend au moins un autre joint, dans lequel ledit au moins un autre joint fait également partie de la pièce moulée par injection à deux composants.

8. Dispositif d'administration selon la revendication 6, dans lequel l'au moins un autre passage est une traversée pour un élément de commande et/ou un ou plusieurs contacts électriques.

9. Dispositif d'administration selon l'une des revendications précédentes, **caractérisé en ce que** le dispositif d'administration est une pompe à perfusion.

10. Dispositif d'administration selon la revendication 8, **caractérisé en ce que** la pompe à perfusion est une pompe pansement ou une pompe patch.

11. Dispositif d'administration selon l'une des revendications 1 à 7,
**caractérisé en ce que** le dispositif d'administration est un appareil d'injection, en particulier un appareil d'injection en forme de broche.
